# EUROPEAN PATENT SPECIFICATION

(11) **EP 4 551 190 B1**
(45) Date of publication and mention of the grant of the patent: **25.03.2026**
(21) Application number: 23739561.1
(22) Date of filing: 07.07.2023
(51) Int. Cl.: A61K 9/00, A61K 9/70, A61K 31/137, A61P 37/08, A61K 47/32, A61K 47/38, A61K 47/10, A61K 47/34, A61K 47/20

(54) **ORAL THIN FILMS COMPRISING ADRENALINE**
ORALE DÜNNFILME ENTHALTEND ADRENALIN
COUCHES MINCES ORALES COMPRENANT ADRÉNALINE

(30) Priority: 07.07.2022 EP 22183690
(43) Date of publication of application: 14.05.2025
(73) Proprietor: LTS Lohmann Therapie-Systeme AG, 56626 Andernach (DE); ALK-Abelló A/S, 2970 Hørsholm (DK)
(72) Inventor: MÜLLER, Markus, 53840 Troisdorf (DE); LINN, Michael, 55596 Waldböckelheim (DE); RICHTER-FRIIS, Martin, 2970 Hørsholm (DK)
(74) Representative: Meissner Bolte Partnerschaft mbB
(86) International application number: PCT/EP2023/068920
(87) International publication number: WO 2024/008952

(56) References cited:
- WO-A1-2021/001461
- WO-A1-2022/195521
- CN-A- 112 236 133
- US-A1- 2019 022 022

## Description

The present invention is directed to an oral thin film, a process for its preparation and its use as a medicament.

Oral thin films (OTF) are thin films containing at least one pharmaceutically active substance that are placed directly in the oral cavity or applied to the oral mucosa and dissolve there. In particular, these are thin polymer-based films containing active ingredient which, when applied to a mucosa, especially the oral mucosa, release the active ingredient directly into it. The very good blood circulation of the oral mucosa ensures a rapid transfer of the active ingredient into the blood circulation. This dosage system has the advantage that the active ingredient is largely absorbed through the mucosa, thus avoiding the "first pass metabolism" that occurs with the conventional dosage form of an active ingredient in tablet form.

A problem with the administration of adrenaline, especially in dissolved form in an oral thin film, is that the active ingredient gets initially dissolved or partially dissolved in the process of the preparation of the oral thin film and partially recrystallizes when the solvent is evaporated. Recrystallization is undesirable, however, as this causes a morphological change in the active ingredient and can also have a negative effect on the chemical stability of the active ingredient. In particular, an inhomogeneous appearance on the surface can occur, which is shown in spots and/or macroscopically visible crystals/crystal accumulations. In addition, recrystallization can also have a negative effect on the mechanical properties of the oral thin film.

While the degradation of adrenaline to adrenochrome leads to a strong coloration, the degradation mechanisms to D-adrenaline and adrenaline sulfonate are responsible for the quantitatively largest degradation. This happens via a planar sp²-transition state which is stabilized by the activated aromatic. Reactions of the transition state with water lead to the formation of D- and L-adrenaline in a ratio of 1:1, which in the long run leads to a racemization of the starting material. The large use of sodium metabisulfite in the existing market products leads to the reaction of sulfite (SO₃²⁻) with adreanlin (S_{N}1 reaction). At the end of shelf-life, known market products therefore contain up to 15% adrenaline sulfonate.

US 2018/0125977 A1 discloses pharmaceutical compositions for the administration of adrenaline, wherein the adrenaline is partially dissolved in the composition.

Further oral film formulations are disclosed in WO 2022/195521 A1, WO 2021/001461 A1, US 2019/022022 A1 and CN 112 236 133 A. Foamed film formulations are disclosed in WO 2018/224591 A1.

The problem underlying the present invention is to overcome the aforementioned disadvantages of the prior art. In particular, the problem of the present invention is to provide an oral thin film for the administration of adrenaline or a pharmaceutically acceptable salt thereof, in which uncontrolled recrystallization of the adrenaline or the pharmaceutically acceptable salt thereof is largely prevented. Furthermore, the adrenaline or the pharmaceutically acceptable salt thereof should also be present in the oral thin film in a chemically largely stable state so that the degradation or racemization of the pharmaceutically active L-adrenaline is prevented without the need to use large amounts of antioxidants, in particular sodium metabisulfite. Furthermore, the oral thin film should be easy and inexpensive to produce.

This problem is solved by an oral thin film according to claim 1 which comprises one or more layer(s), wherein at least one layer contains adrenaline or a pharmaceutically acceptable salt thereof and at least one polymer, characterized in that the adrenaline is substantially undissolved in the at least one polymer.

Such a dosage form makes it possible to eliminate the above-mentioned disadvantages. In particular, the adrenaline is crystalline during the entire production process, so that during the production or storage of the oral thin film, uncontrolled or partial recrystallization does not occur, which would result in the above-mentioned disadvantages.

Due to the polymer matrix of the oral thin film, the active ingredient adrenaline is also well protected against oxygen and extrinsic moisture. Since adrenaline in crystalline form is also chemically very stable, this largely prevents the racemization of L-adrenaline, even without having to resort to large amounts of antioxidants, especially sodium metabisulfite.

Substantially undissolved means that more than 95 % by weight, preferably more than 98 % of adrenaline is present undissolved, i.e. crystalline.

Preferably, adrenaline is at least poorly soluble in the at least one polymer at temperatures of 15°C to 25°C. The definition of different grades of solubility is given below.

| Term | g·l-¹ solvent |
|---|---|
| Very easily soluble | >1000 |
| Easily soluble | 100 to 1000 |
| Soluble | 33 to 100 |
| Less soluble | 10 to 33 |
| Poorly soluble | 1 to 10 |
| Hardly soluble | 0.1 to 1 |
| Insoluble | < 0.1 |

The adrenaline is thus preferably present as a solid suspension in the at least one polymer. That is to say that adrenaline is present as particles which are dispersed in the oral film matrix / polymer. As further disclosed herein, an oral thin film may comprise one or more layer(s), wherein at least one layer contains adrenaline or a pharmaceutically acceptable salt thereof and at least one polymer, characterized in that the adrenaline or the pharmaceutically acceptable salt thereof is present in micronized particles, preferably micronized particles having a particle size distribution of d(99) < 10 µm.

Adrenaline or epinephrine is a hormone produced in the adrenal medulla which belongs to the group of catecholamines. Adrenaline is also found in the central nervous system, where it is present as a neurotransmitter in adrenergic nerve cells. Adrenaline mediates its effects by activating G-protein-coupled receptors, the adrenoceptors.

Adrenaline is also known under the systematic IUPAC name 4-[1-hydroxy-2-(methylamino)ethyl]benzene-1,2-diol. Only the L-enantiomer is pharmaceutically active.

The oral thin film according to the invention is therefore characterized in that the adrenaline or the pharmaceutically acceptable salt thereof is present as the L-enantiomer, i.e. the film comprises the L-adrenaline or a pharmaceutically acceptable salt thereof.

The adrenaline is especially preferred as adrenaline hydrogentartrate, especially as L-adrenaline hydrogentartrate, also named L-adrenaline bitartrate. Other feasible salts may be L-adrenaline hydrochloride.

The oral thin film according to the invention is further preferably characterized in that the adrenaline or the pharmaceutically acceptable salt thereof is present in the oral thin film as micronized particles. The micronized particles might be characterized by having a particle size distribution of d(99) < 10 µm. As the particle size distribution is preferably determined by laser diffraction which determined the volume-based particle size distributions, such as dv(99), the micronized particles might have a particle size distribution of dv(99) < 10 µm.

The feature d(99) or dv(99) means that 99% of the particles are smaller than 10 µm. Optionally, the dv(99) may be smaller, such as < 9 µm, < 8 µm, < 5 µm or even in the low micro range such as < 3 µm, < 2 µm, < 1 µm.

The particle size distribution of the adrenaline or the pharmaceutically acceptable salt thereof may be controlled/ determined by laser diffraction technology before being suspended in the at least one polymer.

Alternatively, the size and shape of the adrenaline particles may be determined by light microscopy This method may be used to control the particle sizes of adrenaline or the pharmaceutically acceptable salt thereof also in the oral thin film.

The particle size distribution may also be determined according to European Pharmacopoeia (Ph. Eur.) 10^{th} edition.

The particle size distribution is preferably such that the largest particles do not exceed a size of 12 µm and do not fall below a size of 0.5 µm.

The use of micronized active ingredient on the one hand increases the dissolution rate in the final film, which can be advantageous for the absorption, and on the other hand enables processing into a homogeneous film.

The oral thin film according to the invention is further preferably characterized in that the at least one polymer comprises at least one water-soluble polymer.

Water-soluble polymers comprise chemically different, natural or synthetic polymers whose common characteristic is their solubility in water or aqueous media. A prerequisite is that these polymers have a sufficient number of hydrophilic groups for water solubility and are not cross-linked. The hydrophilic groups can be non-ionic, anionic, cationic and/or zwitterionic.

Preferably the at least one polymer in the oral thin film of the invention is selected from the group consisting of starch and starch derivatives, dextrans, cellulose derivatives such as carboxymethyl cellulose, hydroxypropyl cellulose, hydroxyethyl cellulose, hydroxypropylmethyl cellulose, hydroxypropylethyl cellulose, sodium carboxymethyl cellulose, ethyl or propyl cellulose, polyacrylic acids, polyacrylates, polyvinyl pyrrolidones, polyvinyl alcohols, polyvinyl pyrrolidone/polyvinyl acetate copolymers, polyethylene oxide polymers, polyacrylamides, polyethylene glycols, polyvinyl alcohol/polyethylene glycol copolymers, gelatine, collagen, alginates, pectin, pullulan, tragacanth, chitosan, alginic acid, arabinogalactan, galactomannan, agar, agarose, carrageenan, natural gums and mixtures thereof.

Hydroxypropyl cellulose, polyvinyl pyrrolidones, polyvinyl pyrrolidone/polyvinyl acetate copolymers, polyvinyl alcohol, polyvinyl alcohol/polyethylene glycol copolymers and/or pectin (amylopectin) are particularly preferred.

Most preferred polymers are hydroxypropyl cellulose and polyvinyl pyrrolidone/polyvinyl acetate copolymers.

The oral thin film according to the invention is preferably characterized in that the oral thin film contains less than 1% by weight, preferably less than 0.5% by weight, more preferably less than 0.1% by weight, based on the total weight of the respective layer of the oral thin film or based on the total weight of the oral thin film, respectively, of an antioxidant.

Known antioxidants or stabilizers used include in particular tocopherols and their esters, sesamol from sesame oil, coniferous benzoate from benzoin resin, gallates (including methyl, ethyl, propyl, amyl, butyl, lauryl gallate), butylated hydroxyanisole (BHA/BHT, also butyl-p-cresol), ascorbic acid and salts and esters thereof (e.g. acorbyl palmitate), erythorbic acid (isoascorbinic acid) and salts and esters thereof, monothioglycerol, sodium formaldehyde sulfoxylate, sodium metabisulfite, sodium bisulfite, sodium sulfite, potassium metabisulfite, butyl hydroxyanisole, butyl hydroxytoluene (BHT) and/or propionic acid.

The largely or complete absence of antioxidants has the advantage that the antioxidants cannot react with the active ingredient and produce undesirable compounds. Furthermore, antioxidants can trigger allergic reactions. The absence or a reduction of antioxidants is therefore advantageous.

In particular, it is advantageous that the oral thin film of the invention contains less than 1% by weight, preferably less than 0.5% by weight, more preferably less than 0.1% by weight of a metabisulfite, such as sodium metabisulfite or potassium metabisulfite, based on the total weight of the respective layer of the oral thin film or based on the total weight of the oral thin film, respectively.

Especially a low amount or even the absence of sodium metabisulfite is advantageous, as sodium metabisulfite can cause allergic reactions and can react with adrenaline to form adrenaline sulfonate.

The oral thin film according to the invention is preferably characterized in that the oral thin film contains less than 5% by weight, preferably less than 4% by weight, preferably less than 3% by weight, preferably less than 2% by weight, preferably less than 1% by weight, preferably less than 0.5% by weight and more preferably less than 0.1% by weight of antimicrobial substances, based on the total weight of the respective layer of the oral thin film or based on the total weight of the oral thin film, respectively.

The absence of antimicrobial substances has the advantage that antimicrobial substances may also be toxic to the patient or may cause allergic reactions. This risk is reduced by dispensing with antimicrobial substances as far as possible.

Known antimicrobial substances comprise especially phyto-extracts.

The oral thin film according to the invention is therefore preferably characterized in that the oral thin film contains less than 5% by weight, preferably less than 4% by weight, preferably less than 3% by weight, preferably less than 2% by weight, preferably less than 1% by weight, preferably less than 0.5% by weight and more preferably less than 0.1% by weight of phyto-extract, based on the total weight of the respective layer of the oral thin film or based on the total weight of the oral thin film, respectively.

The oral thin film according to the invention is preferably characterized in that the oral thin film contains less than 10% by weight, preferably less than 9% by weight, preferably less than 8% by weight, preferably less than 7% by weight, preferably less than 6% by weight, preferably less than 5% by weight and more preferably less than 3% by weight of water, based on the total weight of the respective layer of the oral thin film or based on the total weight of the oral thin film, respectively.

In order to determine the water content, the oral thin film is analyzed via coulometric Karl Fischer titration, typically after dissolution in 5 mL of dimethyl sulfoxide through shaking on a laboratory shaker at about 1200 rpm for 10 min. Thus, the water content may be determined by Karl Fischer titration.

The oral thin film according to the invention may however comprise up to 10% by wt., preferably up to 4% by wt. of an organic solvent, such as ethanol and/or acetone, based on the total weight of the respective layer of the oral thin film or based on the total weight of the oral thin film, respectively.

The oral thin film according to the invention is characterized in that the oral thin film contains at least 40% by weight, preferably at least 45% and more preferably at least 50% by weight of adrenaline or a pharmaceutically acceptable salt thereof (as defined above), based on the total weight of the respective layer of the oral thin film or based on the total weight of the oral thin film, respectively.

Preferably, the oral thin film according to the invention is characterized in that the oral thin film contains 40% by weight to 65% by weight, preferably 45% to 60% by weight and more preferably 50% by weight to 56% by weight to of adrenaline or a pharmaceutically acceptable salt thereof (as defined above), based on the total weight of the respective layer of the oral thin film or based on the total weight of the oral thin film, respectively.

The oral thin film of the invention is preferably characterized in that the oral thin film contains at least 30% by weight to 60% by weight, preferably 33% by weight to 55% by weight, preferably 35% by weight to 50% by weight, preferably 38% by weight to 44% by weight of the at least one polymer, based on the total weight of the respective layer of the oral thin film or based on the total weight of the oral thin film, respectively.

Blends of several polymers can also be used. In this case the sum of all polymers used is preferably 30% by weight to 60 % by weight, preferably 35% by weight to 55% by weight, preferably 38% by weight to 51% by weight, preferably 39% by weight to 50 % by weight, based on the total weight of the respective layer of the oral thin film or based on the total weight of the oral thin film, respectively.

The oral thin film according to the invention is preferably characterized in that the oral thin film further comprises at least one excipient selected from the group consisting of dyes, flavors, sweeteners, taste masking agents, emulsifiers, enhancers, pH regulators, humectants and/or preservatives.

The excipients may each be present in the oral thin film in an amount of about 0.001 to 10% by weight, preferably 0.01 to 5% by weight, preferably 0.01 to 2% by weight, based on the total weight of the respective layer of the oral thin film or based on the total weight of the oral thin film, respectively.

The oral thin film according to the invention is further preferably characterized in that the oral thin film has a mass per unit area of 20 to 400 g/m², preferably of 100 to 350 g/m².

The oral thin film according to the invention is preferably characterized in that the oral thin film comprises at least two layers.

The two layers are preferably identical, but can also have a different composition.

It is also possible that the oral thin film according to the invention has more than two layers. Again, the compositions of the individual layers are either identical or variable.

Preferably the oral thin film of the invention is characterized in that the oral thin film comprises at least two layers, wherein the at least two layers each contain adrenaline or a pharmaceutically acceptable salt thereof and at least one polymer, and in that adrenaline is substantially undissolved in each of the at least one polymers.

The oral thin film according to the invention is further preferably characterized in that the multiple layers are joined by a pharmaceutically acceptable adhesive.

The several layers, preferably two layers, can also be joined by other methods familiar to the person skilled in the art. These include, for example, sealing or heat-sealing, direct coating or laminating of the first layer with a second layer.

The oral thin film according to the invention is preferably characterized in that the multiple layers are joined by a pharmaceutically acceptable adhesive, the pharmaceutically acceptable adhesive preferably comprises at least one water-soluble polymer and at least one plasticizer.

The multilayer oral thin film of the invention is further preferably characterized in that the at least one water-soluble polymer in the adhesive comprises shellac, a vinylpyrrolidone/vinyl acetate copolymer, a polyvinylcaprolactam/polyvinyl acetate/polyethylene glycol copolymer, hydroxypropyl cellulose or hydroxypropylmethyl cellulose and/or polyvinylpyrrolidone.

Preferably, the oral thin film according to the invention is characterized in that the at least one plasticizer in the adhesive comprises glycerol, polyethylene glycol, in particular polyethylene glycol 200, sorbitol and/or tributyl citrate.

In particular, the at least one plasticizer in the adhesive comprises glycerol, polyethylene glycol 200 and/or tributyl citrate.

The use, preferably as a mixture, of at least one water-soluble polymer and at least one plasticizer produces a sticky mixture, which, preferably after drying, can be used as an adhesive layer in the oral thin film according to the invention.

Preferably the weight ratio of the at least one water-soluble polymer to the at least one plasticizer in the adhesive is about 85 to 50 to about 15 to 50, preferably 85 to 65 to about 15 to 35, more preferably about 80 to 60 to about 20 to 40, more preferably about 80 to 50 to about 20 to 50, more preferably about 82 to 68 to about 18 to 32 and most preferably about 80 to 70 to about 20 to 30.

If too little or too much plasticizer is used, the mixture is either not sticky or a processing of the mixture is not possible.

It has been shown that even special mixtures of two polymers are already so tacky that they can be used as adhesives. In this case, a mixture of 5 to 15% by weight of polyvinylpyrrolidone/polyvinyl acetate copolymer and 35 to 45% by weight of hydroxypropyl cellulose has proven to be useful.

The present invention also concerns a process for producing the oral thin film according to the invention, as disclosed in claim 9.

Analogously, additional layers can of course be applied to obtain an oral thin film with any number of layers.

All preferred embodiments described above for the oral thin film apply analogously to the methods according to the invention.

The solvent in which adrenaline or the pharmaceutically acceptable salt thereof is substantially insoluble, comprises ethanol, ethyl acetate, methanol, propanol and/or isopropanol.

These solvents have the advantage that the common polymers are well soluble in them, but adrenaline is essentially not.

Essentially insoluble is understood to mean a solubility of at least poorly soluble in the at least one polymer at temperatures of 15°C to 25°C as defined in the 4 table above.

The present invention is also directed to an oral thin film obtainable by the aforementioned method.

All preferred embodiments described above for the oral thin film and the method for obtaining the oral thin film apply analogously to the oral thin film obtainable by the method according to the invention.

The present invention is further directed at an oral thin film, as described above or obtainable by the above-mentioned method, for use as a medicament, in particular in the treatment of allergic reactions, in the treatment of shock conditions, in the use in emergency medicine, e.g. for resuscitation, treatment of respiratory diseases and/or for local vasoconstriction.

The invention is further explained by means of non-restrictive examples.

### Examples

### Example 1:

An oral thin film comprising micronized adrenaline was prepared with the excipients listed in Table 1.

**Table 1:**

| Material | Function | Amount [wt.-%] |
|---|---|---|
| Adrenaline-hydrogentartrate* | Active ingredient | 50.0 |
| Kollidon VA 64¹ | Polymer | 39.9 |
| HPC² | Polymer | 10.0 |
| Sodium metabisulfite | Stabilizer/antioxidant | 0.1 |

| | | |
|---|---|---|
| ¹: Polyvinylpyrrolidone/polyvinyl acetate copolymer (from BASF) ^{2:} Hydroxypropyl cellulose (from Ashland) *: Adrenaline bitartrate had a particle size distribution with D(99)=8.76 µm | | |

HPC was dissolved in ethanol as process solvent. Subsequently, Kollidon VA 64, sodium metabisulfite and adrenaline hydrogentartrate were added.

This suspension was applied to a siliconized liner and dried.

An adhesive of the composition listed in Table 2 was also prepared.

**Table 2:**

| Material | Function | Amount [wt.-%] |
|---|---|---|
| Kollidon VA 64 | Polymer | 80.0 |
| Glycerol | Polymer | 20.0 |

For this purpose Kollidon VA 64 was dissolved in ethanol as process solvent. Then glycerol was added.

The coating was applied on a siliconized liner and then dried to obtain an adhesive layer.

A multilayer oral thin film was then produced. For this purpose, a laminate piece of the adhesive layer was applied to a laminate piece of the active ingredient layer and another laminate piece of the active ingredient layer was applied to the other side of the adhesive layer.

Advantages of this multilayer oral thin film include better stability of the adrenaline (see also Example 2), no acetate odour, which often occurs in formulations with dissolved adrenaline, and no uncontrolled recrystallization.

### Example 2:

Single-layer oral thin film 197Adr0100 was prepared analogous to Example 1.

Formulations 197Adr0086, 197Adr0078, 197Adr0091 and 197Adr0084 were prepared as follows:
The production of the oral thin films can be carried out by techniques of mass production known to the person skilled in the art, e.g. by agitating/mixing the contained components by means of agitator motor and suitable stirring tools.

Gas is then added to the resulting mass by stirring, e.g. using a foam whipping machine. The foamy mass may then be coated in a constant layer thickness on a coating carrier by suitable equipment (roller applicator, doctor blade, coating box, etc.). The resulting foam comprises cavities.

All temperature-stable web-shaped materials from which the dry film can be removed can serve as coating carrier. This can be achieved by material selection of the coating carrier (different surface tensions between foamed mass and substrate) or by suitable non-adhesive coatings of the coating carrier with e.g. silicones or fluoropolymers.

The process solvent(s) contained, usually water or mixtures of water and organic, water-miscible solvents, is/are removed by drying. From the solid foam layer thus obtained, the oral thin films can be cut or punched to the appropriate size.

**Table 3:**

| **Material** | **197Adr0086** | **197Adr0078** | **197Adr0091** | **197Adr0084** | **197Adr0100** |
|---|---|---|---|---|---|
| Name | Kollicoat | PVA | Kollicoat + Phentolamin | Kollicoat + Proloc | Suspension |
| Adrenaline hydrogentartrate* | 55.00% | 55.00% | 55.00% | 55.00% | 50.00% |
| Kollicoat IR¹ | 43.90% | - | 38.80% | 38.90% | - |
| PVA 4-88² | - | 39.90% | - | - | - |
| Kollidone VA64³ | - | - | - | - | 39.90% |
| HPC4 | - | - | - | - | 10.00% |
| Proloc 15⁵ | - | - | - | 5.00% | - |
| Phentolamine | - | - | 5.10% | - | - |
| Glycerol | 1.00% | 5.00% | 1.00% | 1.00% | - |
| Triacetine | - | - | - | - | - |
| Na-metabisulfit | 0.10% | 0.10% | 0.10% | 0.10% | 0.10% |
| Solvent | Aqua purificata | Aqua purificata | Aqua purificata | Aqua purificata | Ethanol |
| Area weight (dry) | 294 g/m² | 294 g/m² | 294 g/m² | 294 g/m² | 323.4 g/m² |
| | | | | | |
| Adrenaline hydrogentartrate mg / OTF size | 72.8 mg / 4.5 cm² | 72.8 mg / 4.5 cm² | 72.8 mg / 4.5 cm² | 72.8 mg / 4.5 cm² | 72.8 mg / 4.5 cm² |

| | | | | | |
|---|---|---|---|---|---|
| ^{1:} Polyvinyl alcohol/polyethylene glycol copolymer (from BASF) ^{2:} Polyvinyl alcohol 4-88 (from Merck) ^{3:} Polyvinylpyrrolidone/polyvinyl acetate copolymer (from BASF) ^{4:} Hydroxypropyl cellulose (from Ashland) ^{5:} Carbomer / Amylopectin (from Henkel) *: Adrenaline bitartrate had a particle size distribution with D(99)=8.76 µm | | | | | |

The formulation 197Adr0100 corresponds to an oral thin film according to the invention.

The other formulations correspond to prototype formulations in which the active ingredient is partially dissolved.

The degradation of L-adrenaline and the racemization into D-adrenaline after storage at 25°C and 40°C, respectively were measured by HPLC. The results are summarized in Tables 4 and 5. The measurement was performed by HPLC and by comparing the relative peak sized of the peak arising from the L- and the D-enantiomer, respectively.

**Table 4:**

| Storage at 25°C | | | | | | | | | |
|---|---|---|---|---|---|---|---|---|---|
| | | T = 0 | | T = 6 weeks | | T = 3 months | | T = 5 months | |
| | | Ret.Time | Rel.Area | Ret.Time | Rel.Area | Ret.Time | Rel.Area | Ret.Time | Rel.Area |
| | | min | % | min | % | min | % | Min | % |
| 197Adr0100 | L-adr | 23.9 | 99.35 | 16.6 | 99.26 | 17.8 | 99.28 | 17.5 | 99.26 |
| | D-adr | 26.7 | 0.65 | 18.3 | 0.68 | 19.5 | 0.71 | 19.3 | 0.68 |
| 197Adr0086 | L-adr | 23.8 | 99.28 | 16.6 | 98.63 | 17.7 | 98.58 | 17.5 | 98.75 |
| | D-adr | 26.7 | 0.73 | 18.3 | 0.81 | 19.5 | 0.91 | 19.3 | 0.89 |
| | Unknown impurity | | | 29.6 | 0.43 | 33.8 | 0.35 | 32.8 | 0.25 |
| | | | | | | | | | |
| 197Adr0078 | Unknown impurity | | | 12.6 | 0.22 | 12 | 0.15 | | |
| | L-adr | 23.9 | 98.85 | 16.6 | 97.35 | 17.8 | 98.61 | 17.5 | 98.60 |
| | D-adr | 26.7 | 1.15 | 18.3 | 1.34 | 19.5 | 1.03 | 19.4 | 1.07 |
| | Unknown impurity | | | 29.6 | 0.92 | 33.9 | 0.24 | 33.0 | 0.21 |
| | | | | | | | | | |
| 197Adr0084 | L-adr | 23.9 | 99.24 | 16.6 | 98.75 | 17.8 | 98.66 | 17.6 | 98.69 |
| | D-adr | 26.8 | 0.77 | 18.3 | 0.82 | 19.5 | 0.95 | 19.3 | 0.96 |
| | Unknown impurity | | | | | 33.9 | 0.32 | 33.0 | 0.24 |

**Table 5:**

| Batch no. | Formulation | | | Storage at 40°C | | | | | | | | | |
|---|---|---|---|---|---|---|---|---|---|---|---|---|---|
| | | | | T = 0 | | T = 2 weeks | | T = 6 weeks | | T = 3 months | | T = 5 months | |
| | | | | Ret.Time | Rel.Area | Ret.Time | Rel.Area | Ret.Time | Rel.Area | Ret.Time | Rel.Area | Ret.Time | Rel.Area |
| | | | | min | % | min | % | min | % | min | % | min | % |
| 197Adr0100 | Suspension | 71 | UI | | | | | | | | | 13.3 | 0.06 |
| | | | L-adr | 23.9 | 99.35 | 24.3 | 99.38 | 16.6 | 99.26 | 17.8 | 99.20 | 17.6 | 99.26 |
| | | | D-adr | 26.7 | 0.65 | 27.2 | 0.62 | 18.3 | 0.66 | 19.6 | 0.72 | 19.4 | 0.68 |
| 197Adr0086 | Kollicoat | 55 | UI | | | | | 12.6 | 0.11 | | | | |
| | | | L-adr | 23.8 | 99.28 | 24.3 | 99.02 | 16.6 | 98.41 | 17.8 | 98.33 | 17.6 | 97.96 |
| | | | D-adr | 26.7 | 0.73 | 27.2 | 0.86 | 18.3 | 1.06 | 19.6 | 1.3 | 19.4 | 1.56 |
| | | | UI | | | | | 29.7 | 0.28 | 34.2 | 0.18 | 33.4 | 0.21 |
| 197Adr0078 | PVA | 53 | UI | | | 10.6 | 0.12 | | | | | | |
| | | | UI | | | 18 | 0.15 | 12.9 | 0.13 | 12.1 | 0.15 | 11.8 | 0.20 |
| | | | L-adr | 23.9 | 98.85 | 24.2 | 98.30 | 16.6 | 97.73 | 17.9 | 97.62 | 17.7 | 96.96 |
| | | | D-adr | 26.7 | 1.15 | 27.2 | 1.40 | 18.3 | 1.58 | 19.6 | 1.89 | 19.4 | 2.33 |
| | | | UI | | | | | 29.7 | 0.31 | 32 | 0.12 | 31.7 | 0.11 |
| | | | UI | | | | | | | 34 | 0.16 | 33.4 | 0.20 |
| 197Adr0084 | Proloc + kollicoat | 54 | | | | | | | | | | 11.8 | 0.11 |
| | | | L-adr | 23.9 | 99.24 | 24.3 | 99.04 | 16.6 | 98.57 | 17.9 | 98.10 | 17.7 | 97.45 |
| | | | D-adr | 26.8 | 0.77 | 27.2 | 0.87 | 18.3 | 1.09 | 19.6 | 1.58 | 19.4 | 2.04 |
| | | | UI | | | | | 29.8 | 0.22 | 34.3 | 0.14 | 33.5 | 0.19 |

| | | | | | | | | | | | | | |
|---|---|---|---|---|---|---|---|---|---|---|---|---|---|
| UI= unknown impurity | | | | | | | | | | | | | |

Storing the OTF at 40°C in a sealed aluminum pouch, the quantity of adrenaline is only slightly decreased after 5 months (values indicate change with respect to initial value)
197Adr0100: 0.09% reduction in adrenaline, of which 0.03% racemization
197Adr0086: 1.32% reduction in adrenaline, of which 0.83% is racemization
197Adr0078: 1.89% reduction in adrenaline, of which 1.18% racemization
197Adr0084: 1.79% reduction in adrenaline, of which 1.27% is racemization

It is shown that the degradation and racemization is lowest with the OTF according to the invention.

## Claims

1. Oral thin film comprising one or more layer(s), wherein at least one layer contains adrenaline or a pharmaceutically acceptable salt thereof and at least one polymer, **characterized in that** more than 95 % by weight of the adrenaline is present undissolved in the at least one polymer, wherein the adrenaline or the pharmaceutically acceptable salt thereof is present as L-enantiomer and the oral thin film contains at least 40% by weight of adrenaline or a pharmaceutically acceptable salt thereof, based on the total weight of the respective layer of the oral thin film or based on the total weight of the oral thin film, respectively.

2. Oral thin film according to claim 1, **characterized in that** the adrenaline or the pharmaceutically acceptable salt thereof is present micronized having a particle size distribution of d(99) < 10 µm.

3. Oral thin film according to any of the preceding claims, **characterized in that** the at least one polymer is selected from the group consisting of starch and starch derivatives, dextrans, cellulose derivatives such as carboxymethyl cellulose, hydroxypropyl cellulose, hydroxyethyl cellulose, hydroxypropylmethyl cellulose, hydroxypropyl ethyl cellulose, sodium carboxymethyl cellulose, ethyl or propyl cellulose, polyacrylic acids, polyacrylates, polyvinylpyrrolidones, polyvinyl alcohols, polyvinylpyrrolidone/polyvinyl acetate copolymers, polyethylene oxide polymers, polyacrylamides, polyethylene glycols, polyvinyl alcohol/polyethylene glycol copolymers, gelatine, collagen, alginates, pectin, pullulan, tragacanth, chitosan, alginic acid, arabinogalactan, galactomannan, agar, agarose, carrageenan, natural gums and mixtures thereof.

4. Oral thin film according to any of the preceding claims, **characterized in that** the oral thin film contains less than 1% by weight of antioxidants, in particular less than 1% by weight of a metabisulfite, based on the total weight of the respective layer of the oral thin film or based on the total weight of the oral thin film, respectively.

5. Oral thin film according to any of the preceding claims, **characterized in that** the oral thin film contains at least 35% by weight of said at least one polymer, based on the total weight of the oral thin film.

6. Oral thin film according to any of the preceding claims, **characterized in that** the oral thin film further comprises at least one excipient selected from the group consisting of dyes, flavors, sweeteners, taste masking agents, emulsifiers, enhancers, pH regulators, humectants and/or preservatives.

7. Oral thin film according to any of the preceding claims, **characterized in that** the oral thin film comprises at least two layers, wherein the at least two layers each contain adrenaline or a pharmaceutically acceptable salt thereof and at least one polymer, **characterized in that** more than 95 % by weight of the adrenaline is present undissolved in each of the at least one polymers.

8. Oral thin film according to any of the preceding claims, **characterized in that** the multiple layers are joined by a pharmaceutically acceptable adhesive, wherein the pharmaceutically acceptable adhesive preferably comprising at least one water-soluble polymer and at least one plasticizer.

9. Method for preparing an oral thin film according to any one of claims 1 to 8, comprising the steps:
a) Preparing a solution comprising said at least one polymer and a solvent comprising ethanol, ethyl acetate, methanol, propanol and/or isopropanol,
(b) addition of adrenaline or a pharmaceutically acceptable salt thereof,
(c) spreading and drying the suspension obtained in step (b), in order to obtain a single-layer oral thin film, and
d) optionally coating the first layer obtained in c) with a pharmaceutically acceptable adhesive and applying a further, preferably a second layer identical to the first, in order to obtain a two-layer oral thin film.

10. Oral thin film according to any one of claims 1 to 8 for use as a medicament.

11. Oral thin film according to any one of claims 1 to 8 for use in the treatment of allergic reactions.

## Patentansprüche

1. Oraler Dünnfilm, umfassend eine oder mehrere Schicht(en), wobei wenigstens eine Schicht Adrenalin oder ein pharmazeutisch unbedenkliches Salz davon und wenigstens ein Polymer enthält, **dadurch gekennzeichnet, dass** mehr als 95 Gew.-% des Adrenalins ungelöst in dem wenigstens einen Polymer vorhanden sind, wobei das Adrenalin oder das pharmazeutisch unbedenkliche Salz davon als L-Enantiomer vorliegt und der orale Dünnfilm wenigstens 40 Gew.-% Adrenalin oder das pharmazeutisch unbedenkliche Salz davon enthält, basierend auf dem Gesamtgewicht der jeweiligen Schicht des oralen Dünnfilms bzw. basierend auf dem Gesamtgewicht des oralen Dünnfilms.

2. Oraler Dünnfilm nach Anspruch 1, **dadurch gekennzeichnet, dass** das Adrenalin oder das pharmazeutisch unbedenkliche Salz davon mikronisiert mit einer Partikelgrößenverteilung von d(99) < 10 µm vorliegt.

3. Oraler Dünnfilm nach einem der vorstehenden Ansprüche, **dadurch gekennzeichnet, dass** wenigstens ein Polymer ausgewählt ist aus der Gruppe, bestehend aus Stärke und Stärkederivaten, Dextranen, Cellulosederivaten wie etwa Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäuren, Polyacrylaten, Polyvinylpyrrolidonen, Polyvinylalkoholen, Polyvinylpyrrolidon-Polyvinylacetat-Copolymeren, Polyethylenoxidpolymeren, Polyacrylamiden, Polyethylenglykolen, Polyvinylalkohol-Polyethylenglykol-Copolymeren, Gelatine, Kollagen, Alginaten, Pektin, Pullulan, Traganth, Chitosan, Alginsäure, Arabinogalactan, Galactomannan, Agar, Agarose, Carrageen, natürlichen Gummis und/oder Mischungen davon.

4. Oraler Dünnfilm nach einem der vorstehenden Ansprüche, **dadurch gekennzeichnet, dass** der orale Dünnfilm weniger als 1 Gew.-% Antioxidantien, insbesondere weniger als 1 Gew.-% Metabisulfit enthält, basierend auf dem Gesamtgewicht der jeweiligen Schicht des oralen Dünnfilms bzw. basierend auf dem Gesamtgewicht des oralen Dünnfilms.

5. Oraler Dünnfilm nach einem der vorstehenden Ansprüche, **dadurch gekennzeichnet, dass** der orale Dünnfilm wenigstens 35 Gew.-% des wenigstens einen Polymers enthält, basierend auf dem Gesamtgewicht des oralen Dünnfilms.

6. Oraler Dünnfilm nach einem der vorstehenden Ansprüche, **dadurch gekennzeichnet, dass** der orale Dünnfilm ferner wenigstens einen Hilfsstoff enthält, der ausgewählt ist aus der Gruppe, bestehend aus Farbstoffen, Geschmacksstoffen, Süßungsmitteln, Geschmacksmaskierungsmitteln, Emulgatoren, Verstärkern, pH-Wert-Regulatoren, Feuchthaltemitteln und/oder Konservierungsmitteln.

7. Oraler Dünnfilm nach einem der vorstehenden Ansprüche, **dadurch gekennzeichnet, dass** der orale Dünnfilm wenigstens zwei Schichten umfasst, wobei die wenigstens zwei Schichten jeweils Adrenalin oder ein pharmazeutisch unbedenkliches Salz davon und wenigstens ein Polymer enthalten, **dadurch gekennzeichnet, dass** mehr als 95 Gew.-% des Adrenalins ungelöst in jedem des wenigstens einen Polymers vorhanden sind.

8. Oraler Dünnfilm nach einem der vorstehenden Ansprüche, **dadurch gekennzeichnet, dass** die mehreren Schichten durch einen pharmazeutisch unbedenklichen Klebstoff verbunden sind, wobei der pharmazeutisch unbedenkliche Klebstoff vorzugsweise wenigstens ein wasserlösliches Polymer und wenigstens einen Weichmacher umfasst.

9. Verfahren zum Herstellen eines oralen Dünnfilms nach einem der Ansprüche 1 bis 8, umfassend die folgenden Schritte:
a) Herstellen einer Lösung, umfassend wenigstens ein Polymer und ein Lösungsmittel, das Ethanol, Ethylacetat, Methanol, Propanol und/oder Isopropanol umfasst,
(b) Zugabe von Adrenalin oder eines pharmazeutisch unbedenklichen Salzes davon,
(c) Verteilen und Trocknen der in Schritt (b) erhaltenen Suspension, um einen einschichtigen oralen Dünnfilm zu erhalten, und
d) gegebenenfalls Beschichten der in c) erhaltenen ersten Schicht mit einem pharmazeutisch unbedenklichen Klebstoff und Aufbringen einer weiteren, vorzugsweise einer zur ersten Schicht identischen zweiten Schicht, um einen zweischichtigen oralen Dünnfilm zu erhalten.

10. Oraler Dünnfilm nach einem der Ansprüche 1 bis 8 zur Verwendung als Medikament.

11. Oraler Dünnfilm nach einem der Ansprüche 1 bis 8 zur Verwendung bei der Behandlung von allergischen Reaktionen.

## Revendications

1. Film mince oral comprenant une ou plusieurs couches, dans lequel au moins une couche contient de l'adrénaline ou un sel pharmaceutiquement acceptable de celle-ci et au moins un polymère, **caractérisé en ce que** plus de 95 % en poids de l'adrénaline est présente dans un état non dissous dans l'au moins un polymère, dans lequel l'adrénaline, ou le sel pharmaceutiquement acceptable de celle-ci, est présente sous forme de L-énantiomère et le film mince oral contient au moins 40 % en poids d'adrénaline ou d'un sel pharmaceutiquement acceptable de celle-ci, sur la base du poids total de la couche respective du film mince oral ou sur la base du poids total du film mince oral, respectivement.

2. Film mince oral selon la revendication 1, **caractérisé en ce que** l'adrénaline, ou le sel pharmaceutiquement acceptable de celle-ci, est présente dans un état micronisé ayant une distribution de taille de particule de d(99) < 10 µm.

3. Film mince oral selon l'une quelconque des revendications précédentes, **caractérisé en ce que** l'au moins un polymère est choisi dans le groupe consistant en amidon et dérivés d'amidon, dextranes, dérivés de cellulose tels que carboxyméthylcellulose, hydroxypropylcellulose, hydroxyéthylcellulose, hydroxypropylméthylcellulose, hydroxypropyléthylcellulose, carboxyméthylcellulose de sodium, éthylcellulose ou propylcellulose, acides polyacryliques, polyacrylates, polyvinylpyrrolidones, alcools polyvinyliques, copolymères de polyvinylpyrrolidone/acétate de polyvinyle, polymères d'oxyde de polyéthylène, polyacrylamides, polyéthylène glycols, copolymères d'alcool polyvinylique/polyéthylène glycol, gélatine, collagène, alginates, pectine, pullulane, tragacanthe, chitosane, acide alginique, arabinogalactane, galactomannane, agar, agarose, carraghénane, gommes naturelles et mélanges de ceux-ci.

4. Film mince oral selon l'une quelconque des revendications précédentes, **caractérisé en ce que** le film mince oral contient moins de 1 % en poids d'antioxydants, en particulier moins de 1 % en poids d'un métabisulfite, sur la base du poids total de la couche respective du film mince oral ou sur la base du poids total du film mince oral, respectivement.

5. Film mince oral selon l'une quelconque des revendications précédentes, **caractérisé en ce que** le film mince oral contient au moins 35 % en poids dudit au moins un polymère, sur la base du poids total du film mince oral.

6. Film mince oral selon l'une quelconque des revendications précédentes, **caractérisé en ce que** le film mince oral comprend en outre au moins un excipient choisi dans le groupe consistant en teintures, arômes, édulcorants, agents de masquage du goût, émulsifiants, exhausteurs, régulateurs de pH, humectants et/ou conservateurs.

7. Film mince oral selon l'une quelconque des revendications précédentes, **caractérisé en ce que** le film mince oral comprend au moins deux couches, dans lequel les au moins deux couches contiennent chacune de l'adrénaline ou un sel pharmaceutiquement acceptable de celle-ci et au moins un polymère, **caractérisé en ce que** plus de 95 % en poids de l'adrénaline est présente dans un état non dissous dans chacun des au moins un polymères.

8. Film mince oral selon l'une quelconque des revendications précédentes, **caractérisé en ce que** les multiples couches sont assemblées par un adhésif pharmaceutiquement acceptable, dans lequel l'adhésif pharmaceutiquement acceptable comprend de préférence au moins un polymère soluble dans l'eau et au moins un plastifiant.

9. Procédé pour préparer un film mince oral selon l'une quelconque des revendications 1 à 8, comprenant les étapes :
a) la préparation d'une solution comprenant ledit au moins un polymère et un solvant comprenant de l'éthanol, de l'acétate d'éthyle, du méthanol, du propanol et/ou de l'isopropanol,
(b) l'ajout d'adrénaline ou d'un sel pharmaceutiquement acceptable de celle-ci :
(c) l'étalement et le séchage de la suspension obtenue à l'étape (b), afin d'obtenir un film mince oral mono-couche, et
d) le revêtement facultatif de la première couche obtenue à l'étape c) à l'aide d'un adhésif pharmaceutiquement acceptable et l'application d'une autre couche, de préférence une seconde couche identique à la première, afin d'obtenir un film mince oral à deux couches.

10. Film mince oral selon l'une quelconque des revendications 1 à 8 pour une utilisation en tant que médicament.

11. Film mince oral selon l'une quelconque des revendications 1 à 8 pour une utilisation dans le traitement de réactions allergiques.
